# EUROPÄISCHE PATENTSCHRIFT

(11) **EP 2 585 132 B1**
(45) Veröffentlichungstag und Bekanntmachung des Hinweises auf die Patenterteilung: **22.04.2015**
(21) Anmeldenummer: 11736265.7
(22) Anmeldetag: 22.06.2011
(51) Int. Cl.: A61M 1/34, A61M 1/36, B01D 15/00, B01J 20/26, B01J 20/28, B01J 20/32

(54) **NEUARTIGES SORPTIONSMITTEL FÜR ENDOTOXINE**
NOVEL SORBENT FOR ENDOTOXINS
NOUVEL AGENT DE SORPTION POUR ENDOTOXINES

(30) Priorität: 24.06.2010 AT 10732010
(43) Veröffentlichungstag der Anmeldung: 01.05.2013
(73) Patentinhaber: Fresenius Medical Care Deutschland GmbH, 61352 Bad Homburg (DE)
(72) Erfinder: FALKENHAGEN, Dieter, A-3500 Krems (AT); HARM, Stephan, A-3508 Krustetten (AT); HARTMANN, Jens, A-3511 Furth (AT); WEBER, Viktoria, A-3500 Krems (AT)
(74) Vertreter: Patentanwaltskanzlei Matschnig & Forsthuber OG
(86) Internationale Anmeldenummer: PCT/AT2011/000273
(87) Internationale Veröffentlichungsnummer: WO 2011/160149

(56) Entgegenhaltungen:
- WO-A1-95/04559
- WO-A2-2010/083545
- US-A- 5 510 242
- BRANDL M ET AL: "Particle Transfer and Detection in a Microspheres Based Detoxification System", BIOCOMPUTATION, BIOINFORMATICS, AND BIOMEDICAL TECHNOLOGIES, 2008. BIOTECHNO '08. INTERNATIONAL CONFERENCE ON, IEEE, PISCATAWAY, NJ, USA, 29. Juni 2008 (2008-06-29), Seiten 120-124, XP031284095, ISBN: 978-0-7695-3191-5

## Beschreibung

Die Erfindung betrifft ein mit Polymyxin beschichtetes Sorptionsmittel zum Entfernen von Endotoxinen aus einer biologischen Flüssigkeit.

Endotoxine sind Lipopolysaccharide (LPS) in der Zellwand gramnegativer Bakterien und werden durch Zelllyse freigesetzt. In der Tat sind Lipopolysaccharide der häufigste Lipidbestandteil der äußeren Zellmembran gramnegativer Bakterien. Endotoxine sind pyrogene Substanzen, d. h. das betroffene Individuum reagiert mit einer starken Entzündungsreaktion und Fieber, wenn Endotoxine, beispielsweise im Zuge einer mikrobiellen Vergiftung, in den Körper gelangen und systemische Wirkung zeigen. Die Anwesenheit von Endotoxinen im Blutkreislauf führt zu einer unkontrollierten Aktivierung der Immunzellen und einem Ungleichgewicht des Gerinnungssystems. In Abhängigkeit ihrer Konzentration können sie zu einer Sepsis führen, welche unter anderem durch hohes Fieber, niedrigen Blutdruck und, in schweren Fällen, durch Multiorganversagen gekennzeichnet ist. Eine Sepsis ist eine sehr ernstzunehmende Erkrankung; die Letalität von Individuen mit schwerer Sepsis oder septischem Schock ist je nach Schweregrad der Erkrankung ca. 30 - 60 %. Auch Patienten mit beeinträchtigter Immunabwehr, wie z. B. Leberpatienten oder Patienten in Chemotherapie, neigen zu bakteriellen Infektionen und zeigen dadurch Symptome einer Endotoxinvergiftung.

Die Struktur eines Lipopolysaccharid-Moleküls ist dreiteilig: Ein Lipid A bildet den Bereich des Moleküls, welcher der Bakterienzelle zugewandt ist; durch das Lipid A wird das Molekül in der äußeren Membran des gramnegativen Bakteriums verankert. Das LPS-Molekül weist ferner eine mittlere, hoch konservierte Kernregion auf, welche an das Lipid A gebunden ist. Der dritte und äußerste Bereich wird durch ein O-spezifisches Polysaccharid (O-Antigen) gebildet, dessen Struktur innerhalb der verschiedenen gramnegativen Bakterien stark variieren kann. Die toxische Wirkung ist auf das Lipid A zurückzuführen, welches erst bei der Zelllyse freigesetzt wird.

Endotoxine können mittels eines geeigneten Sorptionsmittels aus einer mit Endotoxinen verunreinigten biologischen Flüssigkeit wie Blut oder Plasma entfernt werden. Die Behandlung von Patienten mit Endotoxinvergiftungen bzw. Sepsis findet insbesondere im Rahmen einer extrakorporalen Blutreinigung (Apherese) statt.

Aphereseverfahren sind extrakorporal durchgeführte Verfahren, bei welchen pathophysiologisch relevante Blut- und Plasmakomponenten, beispielsweise Biomoleküle wie (Glyko)Proteine, Peptide, Lipide, Lipoproteine und Lipopolysaccharide, aber auch Blutzellen und Blutplasma entfernt werden. Aphereseverfahren können einerseits zu diagnostischen und therapeutischen Zwecken eingesetzt werden, andererseits stellen sie auch eine sehr effektive Möglichkeit dar, bestimmte Blutbestandteile von gesunden Individuen in ausreichender Menge und in ausreichend hoher Reinheit zu gewinnen. Große Bedeutung wird der therapeutischen Apherese zugemessen, da diese bei bestimmten Indikationen oft eine sehr wirkungsvolle und gleichzeitig nebenwirkungsarme Alternative zur medikamentösen Behandlung ist. So kann bei Plasmaphereseverfahren das Plasma entweder vollständig abgetrennt und durch eine Substitutionslösung ersetzt werden, oder es werden nur bestimmte Bestandteile wie LDL, Endotoxine oder Immunglobuline mittels eines Sorptionsmittels daraus entfernt und das Plasma anschließend in den Spender/Patienten wieder zurückgeführt.

Zum Entfernen von Endotoxinen aus einer mit Endotoxinen verunreinigten biologischen Flüssigkeit (zumeist Blut oder Blutplasma) wird diese mit einem Sorptionsmittel, welches sich üblicherweise in einer Sorptionseinrichtung befindet, in engen Kontakt gebracht. Die Endotoxine werden an der Oberfläche des Sorptionsmittels gebunden und aus der biologischen Flüssigkeit entfernt. Die von Endotoxinen befreite biologische Flüssigkeit wird dem Patienten wieder zugeführt. Die Sorptionseinrichtung ist entweder in einem extrakorporalen Blutkreislauf blutseitig oder in einem Plasmakreislauf einer extrakorporalen Blutreinigungsvorrichtung filtratseitig angeordnet Die Endotoxinbindungskapazität und -geschwindigkeit ist dabei von der Beschaffenheit des Sorptionsmittels abhängig.

Die Geschwindigkeit der Endotoxinbindung durch das Sorptionsmittel ist entscheidend für das Überleben des Patienten. Die Zeit, die bleibt, um die Endotoxine aus dem Blut eines Patienten zu entfernen, ist sehr kurz (< 12 h) und kann bei schwerer Sepsis lediglich einige Stunden betragen.

Es hat sich gezeigt, dass Anionenaustauscherharze (z. B. DEAE oder PEI-Gruppen an Cellulose gebunden) sehr gut für die Endotoxin-Bindung geeignet sind. Nachteilig ist jedoch die unerwünschte Bindung wichtiger Faktoren des intrakorporalen Gerinnungssystems wie Protein C und Protein S und die damit verbundene Gerinnungsproblematik.

Diese Gerinnungsproblematik kann durch die Verwendung eines spezifischen Sorptionsmittels, welches immobilisierte Antikörper gegen Endotoxine aufweist, umgangen werden. Diese Möglichkeit ist jedoch aus ökonomischen Gründen nur limitiert anwendbar.

Als sehr nützliche Alternative sind Sorptionsmittel der eingangs genannten Art, bei welchen Polymyxin-Moleküle auf einem wasserunlöslichen, porösen Träger immobilisiert sind, bekannt geworden.

Polymyxine sind antibiotische Substanzen zur Behandlung von Infektionen mit gramnegativen Bakterien. Polymyxine greifen in die Zellwandstruktur ein, indem sie die Permeabilität der Zellmembran erhöhen, aufgrund dessen es zur Zelllyse kommt. Polymyxine binden nicht nur Phospholipide, sondern auch Lipopolysaccharide (Endotoxine) mit hoher Affinität. Aufgrund der neurotoxischen und nephrotoxischen Wirkung der Polymyxine haben nur Polymyxin B und Polymyxin E (Colistin) gewisse therapeutische Bedeutung erlangt. Polymyxin B bzw. Polymyxin E sind daher lediglich für orale oder topische Therapieformen anwendbar. Für eine parenterale, systemische Behandlung von Endotoxinvergiftungen bzw. Sepsis sind sie aufgrund ihrer toxischen Wirkung nicht geeignet.

Aufgrund der neurotoxischen und nephrotoxischen Wirkung des Polymyxins ist es im Sinne der Patientensicherheit bei der klinischen Anwendung eines mit Polymyxin beschichteten Sorptionsmittels wichtig, eine möglichst feste Bindung des Polymyxins an den Träger anzustreben, um eine Verunreinigung des Patientenblutes mit Polymyxin, z. B. durch Ablösen des Polymyxins vom Träger, möglichst gering zu halten oder zu verhindern. Es hat sich daher vermeintlich als notwendig erwiesen, Polymyxin, insbesondere Polymyxin B, auf einem wasserunlöslichen Träger kovalent zu binden und den auf diese Weise mit Polymyxin B beschichteten Träger als Sorptionsmittel zum Entfernen von Endotoxinen aus verunreinigten biologischen Flüssigkeiten einzusetzen.

In der EP 0110 409 A sind Polymyxin B-immobilisierte Träger aus porösem Glas (FPG 2000) sowie Polymyxin B-immobilisierte Polysaccharid-Träger auf Zellulose-Basis (Cellulofine A-3) offenbart Ebenso bekannt sind Mikropartikel aus Zellulose oder derivatisierter Zellulose, an welche Polymyxin B kovalent gebunden ist [Weber V., Loth F., Linsberger I., Falkenhagen D.: Int. J. Artif. Organs 25(7), 679]. Mit Polymyxin beschichteten Zellulose-Trägern konnte eine hohe Endotoxin-Adsorption erreicht werden, jedoch verbunden mit dem Nachteil, dass Polymyxin B an diese Träger mit erheblichem Aufwand kovalent gebunden und die Zellulose folglich vor der Bindung des Polymyxins chemisch aktiviert werden muss.

Die EP 0 129 786 A2 beschreibt ein Endotoxin-Entgiftungsmaterial mit einem faserartigen Träger, an welchem Polymyxin kovalent immobilisiert ist. Der faserartige Träger ist mit funktionellen Gruppen ausgestattet ist, um Polymyxin kovalent an die Oberfläche des Trägers zu binden. Das Endotoxin-Entgiftungsmaterial aus der EP 0129 786 ist als Füllmaterial für ein Adsorptionsmodul (Handelsname: Toraymyxin) [Shoji H. 2003. Extrakorporeal endotoxin removal for the treatment of sepsis: endotoxin adsorption cartridge (Toraymyxin)] auf dem Markt und im Augenblick das einzige Sorptionsmittel, welches für die klinische Behandlung der Sepsis zur Elimination von Endotoxinen im Rahmen einer extrakorporalen Blutreinigung für eine kommerzielle Nutzung zugelassen ist. Ein kritischer Review über die Effektivität von faserartigen Trägern mit immobilisiertem Polymyxin B, in welchem die Qualität der Behandlung als suboptimal dargestellt wird, ist publiziert worden [Cruz DN et al. 2007; Effektiveness of polymyxin B-immobilized fiber column in sepsis: a systematic review. Crit. Care 11(3):137].

Die US 5 510242 A offenbart ein Sorptionsmittel zum Entfernen von Endotoxinen aus wässrigen Lösungen, umfassend einen Träger mit einer hydrophoben Oberfläche, wobei die Oberfläche des Trägers eine adsorptive Beschichtung aus Polymyxin aufweist.

Nachteilig an den bekannten Sorptionsmitteln, die auf einer Bindung der Endotoxine durch Polymyxin beruhen, ist die geringe Endotoxinbindungskapazität- und geschwindigkeit. Da Polymyxin über NH₂-Gruppen am Polymer gebunden ist, ist der Zugang für Endotoxine beeinträchtigt. Weitere Nachteile ergeben sich aus dem aufwändigen und komplizierten Herstellungsverfahren und den damit verbundenen höheren Herstellungskosten.

Obwohl die Letalität von Patienten mit Endotoxinvergiftungen, insbesondere Sepsis, durch die klinische Anwendung des oben genannten Toraymyxin-Adsorptionsmoduls gesenkt werden konnte, ist die Letalität von Patienten mit schwerer Sepsis und septischen Schock trotz maximaler Therapie immer noch sehr hoch. Aus diesem Grund sowie aufgrund der hohen und ansteigenden Inzidenz von septischen Zuständen besteht ein hoher Bedarf an einem Sorptionsmittel mit verbesserter Sorptionsleistung für Endotoxine, welches darüber hinaus den hohen Sicherheitsanforderungen in Bezug auf dessen klinische Anwendung entspricht.

Es ist eine Aufgabe der Erfindung, ein Sorptionsmittel bereitzustellen, mit welchem Endotoxine in hohem Ausmaß und mit hoher Geschwindigkeit aus einer biologischen Flüssigkeit entfernt werden können und welches darüber hinaus kein zusätzliches Gesundheitsrisiko für den Patienten beinhaltet, sondern in der klinischen Anwendung unbedenklich ist.

Die Aufgabe wird durch ein mit Polymyxin beschichtetes Sorptionsmittel zum Entfernen von Endotoxinen aus einer biologischen Flüssigkeit wie eingangs genannt gelöst, welches erfindungsgemäß einen wasserunlöslichen, porösen Träger mit einer neutralen hydrophoben Oberfläche umfasst, wobei die Oberfläche des Trägers eine adsorptive Beschichtung aus Polymyxin und Albumin aufweist, wobei Polymyxin und Albumin nichtkovalent mit der Oberfläche des Trägers verbunden sind.

Dank des erfindungsgemäßen Sorptionsmittels konnte eine deutliche Verbesserung der Endotoxinsorptionskapazität und Endotoxinsorptionsgeschwindigkeit im Vergleich zu Sorptionsmitteln, bei denen Polymyxin kovalent gebunden ist, erreicht werden. Die Erfinder haben in unerwartetem Maße festgestellt, dass - im Vergleich zu den bekannten Sorptionsmitteln - durch das erfindungsgemäße Sorptionsmittel bereits nach einer kurzen Einwirkzeit eine große Endotoxinmenge aus einer Endotoxin-verunreinigten biologischen Flüssigkeit gebunden werden kann. Dies hat große therapeutische Vorteile, insbesondere für Patienten mit Sepsis, da ein großes Volumen an biologischer Flüssigkeit in kurzer Zeit von Endotoxinen befreit werden kann. Durch das erfindungsgemäße Sorptionsmittel können die Überlebenschancen von Patienten mit schwerer Sepsis verbessert werden. Wie bereits erwähnt ist die Geschwindigkeit der Endotoxinbindung durch das Sorptionsmittel entscheidend für das Überleben des Patienten. Auch die Behandlungsdauer im Rahmen einer extrakorporalen Blutreinigung kann dank des erfindungsgemäßen Sorptionsmittels verkürzt werden, wodurch zeitliche, finanzielle und humane Ressourcen eingespart werden können.

Die Erfinder stellten darüber hinaus den überraschenden Sachverhalt fest, dass dank der adsorptiven Beschichtung des Trägers sowohl mit Polymyxin als auch mit Albumin eine Desorption des Polymyxins von der Oberfläche des Trägers im Vergleich zu einem Träger, der nur mit Polymyxin adsorptiv beschichtet wurde, deutlich verringert werden kann. Dank der Erfindung wird deutlich weniger Polymyxin von der Trägeroberfläche desorbiert. Folglich gelangt deutlich weniger toxisches Polymyxin in die biologische Flüssigkeit, insbesondere in das Blut, wodurch die Sicherheit für den Patienten sehr hoch gehalten werden kann. Die zusätzliche Beschichtung mit Albumin führte im Vergleich zu einem Träger, der nur mit Polymyxin adsorptiv beschichtet wurde, zu keiner Beeinträchtigung der Endotoxinsorption durch das Polymyxin.

Viele Adsorber haben, wie zuvor bereits erwähnt, den Nachteil, dass nicht nur die zu entfernenden Substanzen sondern auch physiologisch wichtige Proteine bzw. Faktoren des Blutgerinnungssytems (Antikoagulanzien sowie Prokoagulanzien) in sehr hohem Maße eliminiert werden. Es konnte weiters festgestellt werden, dass wichtige natürliche Gerinnungsfaktoren des Blutes wie Protein C oder dem Blut hinzugefügte Gerinnungshemmer wie Heparin durch das erfindungsgemäße Sorptionsmittel nur geringfügig adsorbiert werden und überraschenderweise konnte sogar eine geringere Adsorption von Heparin und Protein C durch das erfindungsgemäße Sorptionsmittel im Vergleich zu einem nur mit Polymyxin beschichteten Träger beobachtet werden. Das Risiko eines Ungleichgewichts des intrakorporalen Gerinnungssystems bzw. eine unerwünschte Antikoagulation im extrakorporalen bzw. intrakorporalen Blutkreislauf kann dadurch minimiert werden.

Ein weiterer Vorteil der Erfindung gegenüber den bekannten Sorptionsmitteln ist dessen besonders einfache Herstellung. Polymyxin wird im Vergleich zu den aus dem Stand der Technik bekannten Sorptionsmitteln nicht kovalent, sondern - mittels des hydrophoben Abschnitts des Polymyxin-Moleküls - insbesondere über hydrophobe Interaktion an der neutralen, hydrophoben Oberfläche des Trägers immobilisiert. Die Steigerung der Endotoxinbindungseffektivität des erfindungsgemäßen Sorptionsmittels lässt sich möglicherweise dadurch erklären, dass die, insbesondere mittels hydrophober Interaktion vermittelte, Bindung die NH₂-Gruppen des Polymyxins freilässt und diese im Wesentlichen in ihrer Gesamtheit für die Endotoxinbindung zur Verfügung stehen. Es sind keine komplexen chemischen Schritte zum Immobilisieren des Polymyxins an den Träger notwendig. Auch die Bindung des Albumins erfolgt nicht kovalent, sondern ebenfalls über adsorptive Vorgänge. Auch hier sind keine weiteren chemischen Immobilisierungs- oder Vernetzungsschritte oder die Verwendung von Chemikalien, die vor der klinischen Anwendung des Sorptionsmittels wieder rückstandslos entfernt werden müssten, notwendig. Das adsorptive Beschichten von Oberflächen mit Albumin ist bekannt; beispielsweise wird bei Immunassays, z. B. ELISA, Rinderserumalbumin (BSA) eingesetzt, um eine unspezifische Bindung zu verhindern. Die Herstellung des erfindungsgemäßen Sorptionsmittels kann daher ökonomisch, reproduzierbar und ohne Anwendung von zusätzlichen Chemikalien durchgeführt werden.

Unter dem Begriff "adsorptive Beschichtung" aus Polymyxin und Albumin ist demnach zu verstehen, dass Polymyxin und Albumin über adsorptive Vorgänge an der Oberfläche binden und somit nichtkovalent mit der Oberfläche des Trägers verbunden sind. Wie oben bereits erwähnt, ist anzunehmen, dass insbesondere die hydrophobe Interaktion, auch hydrophobe Wechselwirkung genannt, eine wichtige Rolle spielt. Die hydrophobe Interaktion besitzt große biochemische Bedeutung und beruht auf dem Phänomen, dass hydrophobe Moleküle in einer polaren Umgebung zur Assoziation neigen. Die hydrophobe Interaktion ist daher keine Kraft per se, sondern wird durch eine polare Umgebung erzwungen. Bei der vorliegenden Erfindung findet die hydrophobe Interaktion insbesondere zwischen dem hydrophoben Abschnitt des Polymyxin-Moleküls bzw. den hydrophoben Seitenketten des Albumins und den neutralen, hydrophoben inneren und äußeren Oberflächen des porösen Trägers statt. Das adsorptive Beschichten von Oberflächen mit amphiphilen Molekülen ist einem Fachmann bekannt. Beispielsweise beruhen grundlegende chromatographische Abtrennungsverfahren wie zum Beispiel HIC (hydrophobe Interaktionschromatographie) oder die Reversed-Phase Chromatographie auf dem Prinzip der hydrophoben Interaktion, wodurch üblicherweise eine sehr feste Bindung der Moleküle an der Trägeroberfläche erreicht wird. Auch andere nicht-kovalente Interaktionen, einschließlich, ohne Beschränkung, ionischer Bindungen, Wasserstoffbrückenbindungen und van der Waals Wechselwirkungen können bei der Adsorption von Polymyxin und Albumin eine Rolle spielen. Eine mögliche Erklärung für den erfindungsgemäßen Effekt ist, dass die Desorption des Polymyxins durch eine Vielzahl an nichtkovalenten Wechselwirkungen zwischen den Polymyxin- und Albuminmolekülen verringert und dadurch der erfindungsgemäße Effekt bewirkt wird.

Unter dem Begriff "Sorptionsmittel" ist im Rahmen dieser Offenbarung ein Mittel zum Durchführen einer Sorption, vorzugsweise einer Adsorption zu verstehen, d. h. Moleküle, die sich in einer biologischen Flüssigkeit befinden, werden durch die Oberflächenkräfte des Sorptionsmittels festgehalten. In der Beschreibung werden daher anstelle des Begriffs "Sorptionsmittel" auch die Begriffe "Adsorbtionsmittel" bzw. "Adsorbens" bzw. "Adsorber" verwendet. Erfindungsgemäß ist das Sorptionsmittel zur Adsorption von Endotoxinen aus einer mit Endotoxinen verunreinigten, biologischen Flüssigkeit vorgesehen. Anwendung findet das erfindungsgemäße Sorptionsmittel vor allem bei der extrakorporalen Blutreinigung, insbesondere bei Patienten mit septischen Zuständen.

Der hierin verwendete Begriff "Albumin" bezieht sich auf natürlich auftretendes Albumin, insbesondere Humanalbumin. Weiters umfasst der Begriff "Albumin" auch Albuminähnliche Polypeptide, die sich aus Subtypen des Albumins, wie beispielsweise Präalbumin rekrutieren. Die Albumin-ähnlichen Polypeptide können entweder aus natürlichen Quellen stammen oder synthetisch hergestellt werden.

Der im Rahmen der Erfindung verwendete Ausdruck "biologische Flüssigkeit" kann sich auf zellfreie Flüssigkeiten, insbesondere Blutplasma, oder auf Zellen enthaltende Flüssigkeiten, insbesondere Blut, beziehen. Da es im Zuge einer extrakorporalen Blutreinigung auch notwendig ist, andere Flüssigkeiten wie beispielsweise Gerinnungshemmer enthaltende Lösungen (Heparin-Lösung, Citrat-Lösung) oder Substitutiönslösungen (Elektrolyte, Flüssigkeiten zum Ausgleich des Flüssigkeitsverlust) in den extrakorporalen Blutkreislauf bzw. in einen Blutplasmakreislauf einzubringen, ist unter einer biologischen Flüssigkeit auch verdünntes Blut bzw. verdünntes Blutplasma zu verstehen. Die Erfindung ist hauptsächlich für den humanmedizinischen Bereich angedacht und bezieht sich daher auf menschliche biologische Flüssigkeiten. Dies schließt jedoch die Anwendung der Erfindung im veterinärmedizinischen Bereich nicht aus.

Polymyxine sind bekannte chemische Verbindungen, welche ursprünglich aus dem Bakterium *Bacillus polymyxa* stammen. Insbesondere sind Polymyxin B und Polymyxin E (Colistin) als für die vorliegende Erfindung nützliche Polymyxine hervorzuheben.

Der Begriff "wasserunlöslicher, poröser Träger mit einer nicht-ionischen hydrophoben Oberfläche", wie im unabhängigen Anspruch 1 verwendet, bezieht sich im Rahmen dieser Offenbarung auf einen porösen, wasserunlöslichen Feststoff, der äußere und innere Oberflächen aufweist. Die äußeren und inneren Oberflächen sind neutral und hydrophob. Unter dem Begriff "neutral" ist nicht-ionisch zu verstehen. Die Erfindung ist vor allem auf partikelförmige Träger ausgerichtet

Zum Herstellen eines erfindungsgemäßen Sorptionsmittels werden Polymyxin und Albumin in getrennten, aufeinanderfolgenden Beschichtungsschritten am Träger adsorbiert. Dabei hat es sich als zweckmäßig erwiesen, den Träger zuerst mit Polymyxin adsorptiv zu beschichten und anschließend, nach einem Waschschritt zum Entfernen des überschüssigen Polymyxins, die adsorptive Beschichtung mit Albumin durchzuführen. Dadurch ist es möglich, zwischen den beiden Beschichtungsschritten einen Autoklavierschritt einzuführen. Grundsätzlich ist es auch möglich, den Träger zuerst mit Albumin und anschließend mit Polymyxin zu beschichten; allerdings gestaltet sich die sterile Herstellung eines solchen Trägers umständlicher, da die Albuminbeschichtung unter Autoklavierbedingungen denaturiert und folglich alle Schritte mit sterilen, insbesondere sterilfiltrierten, Lösungen durchgeführt werden müssen. Es ist daher von Vorteil, den Träger nach dem adsorptiven Beschichten mit Polymyxin steril zu machen und anschließend die Beschichtung mit Albumin durchzuführen. Sterile Albuminlösungen, die auch für die Herstellung des erfindungsgemäßen Sorptionsmittels geeignet sind, werden häufig eingesetzt und sind kommerziell erhältlich (z. B. Humanes Albumin 20 %, Octapharma, Wien, Österreich).

Ein erfindungsgemäßes Sorptionsmittels wird vorzugsweise mit einem Verfahren, welches auch Teil dieser Erfindung ist, hergestellt, welches die folgenden Schritte umfasst:
a) Bereitstellen eines wasserunlöslichen, porösen Trägers mit einer nicht-ionischen hydrophoben Oberfläche,
b) Adsorptives Beschichten der Oberfläche des Trägers mit Polymyxin durch Inkontaktbringen und Inkubieren des Trägers mit einer wässrigen, Polymyxin enthaltenden Lösung,
c) Waschen des Trägers zum Entfernen von ungebundenem Polymyxin,
d) Adsorptives Beschichten der mit Polymyxin beschichteten Oberfläche des Trägers mit Albumin durch Inkontaktbringen und Inkubieren des Trägers mit einer wässrigen, Albumin enthaltenden Lösung, und
e) gegebenenfalls Waschen des Trägers zum Entfernen von ungebundenem Albumin.

Der Waschschritt e) ist optional und kann daher auch weggelassen werden. Wird der mit Polymyxin und Albumin beschichtete Träger vor der Anwendung für einen längeren Zeitraum zwischengelagert, dann ist es jedoch zweckmäßig, den Schritt e) vor dem Lagern durchzuführen.

Für die Herstellung der wässrigen Polymyxin bzw. Albumin enthaltenden Lösungen werden pharmazeutisch annehmbare Salze verwendet. Pharmazeutisch annehmbare Kationen, wie z. B. Natrium, werden verwendet, um die Ionenstärke zu beeinflussen. In Hinblick auf die Verwendung zur Blutreinigung, bei welcher das Sorptionsmittel direkt in Kontakt mit dem Blut kommt, ist Albumin vorzugsweise in isotonischer Lösung, mehr bevorzugt in isotonischer Kochsalzlösung, gelöst. Geeignete, sterile Albuminlösungen sind, wie oben bereits erwähnt, kommerziell erhältlich. Das Waschen des Trägers beim Herstellungsverfahren zum Entfernen von ungebundenem Polymyxin bzw. Albumin erfolgt bevorzugt ebenso mit einer isotonischen Lösung, insbesondere einer isotonischen Kochsalzlösung.

Um den hohen Anforderungen hinsichtlich der Sterilität des Sorptionsmittels zu entsprechen, ist es zweckmäßig, wenn der Träger zwischen den Schritten b) und c) oder zwischen den Schritten c) und d) autoklaviert wird. Das Autoklavieren erfolgt nach gängiger Praxis, beispielsweise bei 121 ° C für 20 min. Einem Fachmann wird klar sein, dass die nach dem Autoklavieren verwendeten Lösungen, d. h. die wässrige, Albumin enthaltende Lösung und die zum Waschen verwendete Lösung, den klinischen Anforderungen entsprechen sollten, insbesondere, dass sie steril und pyrogenfrei sind.

Für die Praxis ist es besonders zweckmäßig, wenn der Träger ein hydrophobes Polymer ist. Dadurch kann eine gute Reproduzierbarkeit des Trägermaterials gewährleistet werden, insbesondere hinsichtlich der Porosität und der Partikelgröße. Die Porosität und die Partikelgröße lassen sich darüber hinaus sehr gut variieren. Beim hydrophoben Polymer kann es sich sowohl um ein Homopolymer als auch um ein Heteropolymer handeln. Für die praktische Durchführung haben sich vernetzte Polystyrol-Polymere als besonders günstig erwiesen. Bei der extrakorporalen Blutreinigung bestehen hohe Anforderungen an die Sterilität der Vorrichtungsbestandteile, welche mit den Körperflüssigkeiten des Patienten in Kontakt kommen. Dies gilt auch für Sorptionsmittel. Vernetzte Polystyrol-Polymere zeichnen sich durch eine hohe Stabilität gegenüber Hitze und Chemikalien aus und sind in der klinischen Praxis bereits etabliert.

Die Stärke der hydrophoben Interaktion zwischen Polymyxin und Träger ist einerseits durch die Hydrophobizität des neutralen, hydrophoben Trägers und andererseits durch die Ionenstärke des Mediums bestimmt. Wie oben bereits erwähnt wurde, hat Polymyxin neurotoxische und nephrotoxische Wirkung. Es ist daher eine möglichst feste Bindung des Polymyxins an die äußeren und inneren Oberflächen des Trägers erwünscht. Bei einer besonders bevorzugten Variante ist das vernetzte Polystyrol-Polymer ein Polystyrol-Divinylbenzol-Copolymer. Die Oberfläche eines Polystyrol-Divinylbenzol-Copolymers weist eine hohe Hydrophobizität auf, wodurch eine starke Bindung des Polymyxins an die Trägeroberfläche erreicht wird. Es ist natürlich auch möglich, andere neutrale, hydrophobe Polymere hoher Hydrophobizität zu verwenden, welche einem einschlägigen Fachmann gut bekannt sind. Es wurde jedoch festgestellt, dass nach dem Immobilisieren des Polymyxins am neutralen, hydrophoben Polymer immer noch gewisse Mengen an Polymyxin von der Trägeroberfläche desorbieren und in die biologische Flüssigkeit freigesetzt werden. Die Desorption wirkt sich zwar nicht signifikanz auf die Endotoxinsorptionskapazität durch das Polymyxin aus; dennoch können bereits geringe Mengen an Polymyxin neurotoxische und nephrotoxische Wirkung entfalten und somit ein Gesundheitsrisiko für den Patienten darstellen. Die Desorption des Polymyxins kann dank des erfindungsgemäßen Sorptionsmittels, welches neben Polymyxin zusätzlich mit Albumin beschichtet ist, deutlich verringert werden.

Weiters wurde gebunden, dass auch die Porengröße des porösen Trägers hinsichtlich der Endotoxinadsorption von Bedeutung ist. Es ist daher günstig, auch aus Gründen der Reproduzierbarkeit, wenn der poröse Träger eine definierte mittlere Porengröße aufweist. Die mittlere Porengröße des Trägers bezieht sich immer auf jene vor dem Immobilisieren des Polymyxins über hydrophobe Interaktion.

Die mittlere Porengröße kann besonders gut eingestellt werden, wenn der poröse Träger aus einem synthetischen Polymer hergestellt ist. Obwohl einem Fachmann auf dem Gebiet bekannt ist, was unter dem Begriff "mittlere Porengröße" zu verstehen ist und wie die Porösität bzw. die mittlere Porengröße gezielt einstellt werden kann, soll dieser Begriff aus Gründen der Klarheit an dieser Stelle dennoch kurz definiert werden. Die mittlere Porengröße bezieht sich auf den mittleren Durchmesser der Poren. Bei einer gaußschen Größenverteilung der Porendurchmesser eines porösen Materials ist der mittlere Porendurchmesser jener, welchem dem Maximum der Verteilungskurve entspricht. Der mittlere Porendurchmesser kann beispielsweise mittels Stickstoffadsorption (wie beschrieben in Weber et al. 2008; Neutral styrene divinylbenzene copolymers for adsorption of toxins in liver failure. Biomacromolecules 9(4):1322-1328) oder mittels Quecksilberintrusion bestimmt werden. Eingestellt wird die Porengröße eines Polymers durch Variation der Konzentration der beteiligten Monomere bzw. Co-Monomere, des Lösungsmittels oder des Modulators. Je kleiner die Poren des Polymers gewählt sind, umso größer wird die innere Oberfläche des Polymers, die für eine Sorption, insbesondere Adsorption, zur Verfügung steht. Je größer die Poren, umso besser ist die Zugänglichkeit der Poren für größere Moleküle. Ein Herstellungsverfahren für ein synthetisches, hydrophobes Polymer definierter Porengröße, wie es für die Erfindung zur Anwendung kommen kann, ist in der oben genannten Publikation von Weber et al. beschrieben.

Es hat sich herausgestellt, dass eine besonders gute Endotoxinsorption durch das Sorptionsmittel erreicht werden kann, wenn der Träger eine mittlere Porengröße von zumindest 15 nm aufweist. Vorzugsweise weist der Träger eine mittlere Porengröße von zumindest 30 nm auf. Für die klinische Anwendung einer extrakorporalen Blutreinigung ist es jedoch günstig, wenn die mittlere Porengröße des unbeschichteten Trägers nicht größer als 120 nm ist. Die innere Oberfläche des Sorptionsmittels würde ansonsten zu klein werden; die Folge wäre eine Verminderung der Endotoxinsorptionskapazität (Endotoxinadsorptionskapazität). Vorzugsweise weist der Träger daher eine mittlere Porengröße auf, die in einem Bereich von 15nm bis 120 nm gewählt ist.

Mit Vorteil weist der unbeschichtete Träger bei einer Variante eine mittlere Porengröße von ungefähr 30 - 40 nm auf, bei welcher die Eliminierung von Endotoxinen aus einer biologischen Flüssigkeit mit besonders hoher Geschwindigkeit und hoher Effizienz erfolgt. Es ist daher nur eine geringe Menge an Sorptionsmittel notwendig, um eine große Menge Endotoxin zu binden.

Beispielsweise kann die Konzentration des erfindungsgemäßen Sorptionsmittels, wenn als Suspension in einem extrakorporalen Plasmakreislauf angewendet, 1%ig (Vol%) gewählt werden. Ein extrakorporaler Plasmakreislauf, in welchem eine Suspension eines Mikropartikel-förmigen Sorptionsmittels enthalten ist, stellt einen zentralen Bestandteil eines Microspheres-based Detoxification Systems (MDS) dar. Ein MDS ist aus der EP 0776223 B und der US 5855782 bekannt geworden.

Darüber hinaus ist auch die Form des erfindungsgemäßen Sorptionsmittels beim Sorptionsvorgang von Bedeutung. Bei einer vorteilhaften Variante ist das erfindungsgemäße Sorptionsmittel mikropartikelförmig. Die Partikelgröße beeinflusst die Kinetik der Adsorption. Darüber hinaus liegt bei einer kleinen Partikelgröße ein großes Oberflächen/VolumenVerhältnis vor. Bei einer vorteilhaften Untervariante weisen die Mikropartikel eine Partikelgröße von 20 µm oder kleiner auf.

Die Mikropartikel finden insbesondere in einem MDS Anwendung, welches zuvor bereits erwähnt wurde. Die Mikropartikel zirkulieren als Suspension in einem Reinigungskreislauf (Plasmakreislauf) auf der Filtratseite eines Membranfilters. Sollte der Membranfilter allerdings undicht werden, besteht die Gefahr, dass Mikropartikel in den extrakorporalen Blutkreislauf und weiter in den Körper des Patienten gelangen und dort zu einer Lungenembolie führen. Aus diesem Grunde ist es bei einer weiteren Untervariante von Vorteil, wenn die Mikropartikel eine Partikelgröße von 8 µm oder kleiner, idealerweise 5 µm oder kleiner, aufweisen, da bei diesen kleinen Partikelgrößen die Gefahr einer Lungenembolie umgangen werden kann.

Das erfindungsgemäße Sorptionsmittel ist hauptsächlich für die Anwendung in der extrakorporalen Blutreinigung (Apherese) vorgesehen.

In einer wichtigen Anwendungsform der Erfindung kann das Sorptionsmittel als Füllmaterial für eine Sorptionseinrichtung Anwendung finden. Die Sorptionseinrichtung kann als Säule oder Kartusche ausgebildet sein. Je nachdem, welche Blutreinigungsvorrichtung bzw. welches Blutreinigungsverfahren (Hämoperfusion, Plasmapherese/Plasmasorption) angewendet wird, kann die Sorptionseinrichtung blutseitig in einem extrakorporalen Blutkreislauf oder in einem Plasmakreislauf auf der Filtratseite angeordnet sein. Die biologische Flüssigkeit (Blut oder Blutplasma) passiert die Sorptionseinrichtung, wobei die Endotoxine an die immobilisierten Polymyxin-Moleküle des Sorptionsmittels binden. Das gereinigte Blut bzw. Plasma wird dem Patienten wieder zugeführt.

Eine weitere Einsatzmöglichkeit betrifft einen Plasmakreislauf, in welchem das Sorptionsmit tel als Suspension im Plasma verteilt vorliegt. Ein Beispiel für einen solchen Plasmakreislauf findet sich als Vorrichtungselement in einem oben beschriebenen MDS. Das in einem Plasmakreislauf in Suspension vorliegende Sorptionsmittel ist vorzugsweise mikropartikelförmig.

Obwohl das erfindungsgemäße Endotoxin-Sorptionsmittel in erster Linie für die Anwendung in der extrakorporalen Blutreinigung (Apherese) vorgesehen ist, ist ein Einsatz in der Chromatographie ebenfalls denkbar. So kann das Sorptionsmittel als Füllmaterial für Chromatographiesäulen zum Reinigen von Endotoxin-belastetem Blut oder Blutplasma eingesetzt werden. Auch andere Anwendungen zum Entfernen von Endotoxinen aus biologischen Flüssigkeiten oder Wasser sind denkbar.

Das erfindungsgemäße Sorptionsmittel bzw. eine Sorptionseinrichtung enthaltend ein erfindungsgemäßes Sorptionsmittel bzw. ein Plasmakreislauf enthaltend eine Suspension eines erfindungsgemäßen Sorptionsmittels ist besonders zur Behandlung einer Sepsis geeignet.

Die vorliegende Erfindung wird im Folgenden anhand von nicht einschränkenden Beispielen näher erläutert.

### 1. BEISPIEL 1: Herstellung erfindungsgemäßer Sorptionsmittel (Adsorber).

Zum Herstellen erfindungsgemäßer Sorptionsmittel wurden neutrale, hydrophobe Polystyrol-Divinylbenzol-Copolymere zuerst mit Polymyxin B (PMB) und anschließend mit Humanserumalbumin (HSA) adsorptiv beschichtet.

### 1.1. Bereitstellen eines wasserunlöslichen, porösen Trägers mit neutraler, hydrophober Oberfläche

Polystyrol-Divinylbenzol-Copolymere verschiedener Partikelgröße (im Folgenden auch kurz als "Polymer" oder "Träger" oder "unbeschichteter Adsorber" bezeichnet) wurden von der Firma Dow Chemical bezogen und wiesen eine mittlere Porengröße von 30 bis 40 nm auf. Die Polystyrol-Divinylbenzol-Copolymere sind in der Tabelle 1 aufgelistet.

**Tabelle 1: Polystyrol-Divinylbenzol-Copolymere**

| Bezeichnung | Bezeichnung-Hersteller | Partikelgröße [µm] |
|---|---|---|
| #1824 | Microadsorber | 5 |
| HPR10 | Amberchrom® HPR10 | 10 |

Träger #1824 wurde vom Hersteller in Suspension geliefert und 5 x in NaCl gewaschen.

Träger HPR10 wird gemäß den Angaben des Herstellers vorbehandelt.

### 1.2. Adsorptives Beschichten der Polymere mit Polymyxin B (PMB) und Humanserumalbumin (HSA)

Die in Tabelle 1 aufgelisteten Träger #1824 und HPR10 wurden mit PMB und HSA gemäß den folgenden Schritten beschichtet:
a) Für die Beschichtung wird eine entsprechende Menge PMB-Lösung mit einer Konzentration von 5 mg/ml hergestellt. Polymyxin B (PMB) wurde von der Firma Sigma (Kat.Nr.: 81334, Lot: 1348744) bezogen. Für die Beschichtung wurde eine Polymyxin-B-Lösung (PMB-Lösung) hergestellt, wobei 50 mg PMB in 10 ml LAL-Wasser gelöst wurden.
b) Der vorbehandelte Träger wird abzentrifugiert (4000 g, 10 min), der Überstand verworfen und pro ml Trägersediment 5 ml PMB-Lösung für die Beschichtung zupipettiert.
c) Der Träger wird 60min bei 20 ° C mit der PMB-Lösung inkubiert.
d) Nach der Inkubationszeit wird die nunmehr mit PMB beschichtete Adsorbersuspension bei 121 °, 20 min lang in sterilen Glasfläschchen autoklaviert.
e) Anschließend wird der mit PMB beschichtete Adsorber abzentrifugiert (4000 g, 10 min) und 3 mal mit dem 5-fachem Volumen 0,9 % NaCl gewaschen.
f) Der mit PMB beschichtete Adsorber kann als 50%ige Suspension in 0,9 % NaCl im Kühlschrank zwischengelagert werden oder sofort weiter mit HSA beschichtet werden.
g) Für die adsorptive Beschichtung mit HSA wird eine entsprechende Menge 10%ige Humanserumalbumin-Lösung (HSA-Lösung) hergestellt (Bezugsquelle: Humanes Albumin, 20%ig, Octapharma, Wien, Österreich).
h) Der PMB beschichtete und autoklavierte Adsorber wird abzentrifugiert (4000 g, 10 min), der Überstand verworfen und pro ml Adsorbersediment 2,5 ml HSA-Lösung für die Beschichtung zupipettiert.
i) Die Adsorbersuspension wird mindestens 12 h bei 10 ° C mit der HSA-Lösung inkubiert.
j) Nach der Inkubationszeit wird der mit PMB und HSA beschichtete Adsorber 1 mal mit 5 fachem Volumen 0,9%iger NaCl-Lösung gewaschen.
k) Der mit PMB und HSA beschichtete Adsorber wird als 50%ige Suspension in 0,9 % NaCl im Kühlschrank gelagert.

Da fast zur Gänze die zugesetzte PMB Menge zur Beschichtung des Trägers adsorbiert wird, beträgt die PMB-Menge pro g Feuchtadsorber 25 mg.

Die erhaltenen mit PMB und HSA beschichteten erfindungsgemäßen Adsorber sind in der Tabelle 2 zusammengefasst:

**Tabelle 2: Erfindungsgemäße mit PMB und HSA beschichtete Adsorber**

| Bezeichnung des PMB und HSA beschichteten Adsorbers |
|---|
| #1824+PMB+HSA |
| HPR10+PMB+HSA |

### 2. BEISPIEL 2: Desorption von Polymyxin B sowie Endotoxinadsorption - Batchtest

### 2.1. Verwendete Adsorber

Die Desorption des Polymyxin B von der Oberfläche des Adsorbers HPR10+PMB+HSA und die Endotoxinadsorption durch den Adsorber HPR10+PMB+HSA wurden in einem Batchtest untersucht.

Der mit PMB und HSA beschichtete Adsorber HPR10+PMB+HSA (Herstellung siehe Beispiel 1 unter 1.2) wurde beim Polymyxin-B-Desorptionsversuch mit zwei weiteren Adsorbern verglichen, nämlich:
- HPR10+PMB: nur mit PMB beschichteter Träger HPR10. Die Herstellung erfolgte laut den Schritten a) - f) gemäß dem Herstellungsprotokoll in Abschnitt 1.2.
- HPR10+HSA+PMB: Träger HPR10, welcher zuerst mit Albumin und anschließend mit Polymyxin B beschichtet wurde. Die Herstellung dieses Adsorbers, welcher ebenfalls ein erfindungsgemäßer Adsorber ist, erfolgt anhand der im Folgenden angeführten Schritte a) bis j):
   a) Der vorbehandelte HPR10 Träger wird als 50%ige Suspension in 0,9 % NaCl bei 121 ° C 20 min lang autoklaviert.
   b) Nach dem Autoklavieren wird der Träger abzentrifugiert und danach die Albuminbeschichtung durchgeführt.
   c) Für die Beschichtung wird eine entsprechende Menge 10%ige HSA-Lösung (Bezugsquelle: Humanes Albumin, 20%ig, Octapharma, Wien, Österreich) hergestellt und pro ml Trägersediment 2,5 ml HSA-Lösung für die Beschichtung zupipettiert.
   d) Die Trägersuspension wird mindestens 12 h bei 10 ° C mit der HSA-Lösung inkubiert.
   e) Nach der Inkubationszeit wird der HSA-beschichtete HPR10-Adsorber 1 mal mit 5 fachem Volumen 0,9%iger NaCl-Lösung gewaschen und abzentrifugiert (4000 g, 10 min). Der Überstand wird verworfen und anschließend wird die PMB-Beschichtung durchgeführt:
   f) Für die PMB-Beschichtung wird eine entsprechende Menge PMB-Lösung mit einer Konzentration von 5 mg/ml hergestellt. Polymyxin B (PMB) wurde von der Firma Sigma bezogen (Kat.Nr.: 81334, Lot:1348744).
   g) Pro ml Adsorbersediment werden 5 ml PMB-Lösung für die Beschichtung zupipettiert.
   h) Die Adsorbersuspension wird 60 min bei 20 ° C mit der PMB-Lösung inkubiert.
   i) Nach der Inkubationszeit wird die Adsorbersuspension abzentrifugiert (4000 g, 10 min) und 3 mal mit dem 5-fachem Volumen 0,9 % NaCl gewaschen.
   j) Der fertige mit HSA und mit PMB beschichtete HPR10-Adsorber (HPR10+HSA+PMB) wird als 50%ige Suspension in 0,9 % NaCl im Kühlschrank gelagert.

Bei der Untersuchung der Endotoxinadsorption wurde HPR10+PMB+HSA mit HPR10+PMB verglichen. Als Kontrolle wurde ein Teströhrchen ohne Adsorber (Ko o Ads) mitgeführt

### 2.2. Batchtest

1 % (v/v) Adsorber (HPR10+PMB, HPR10+HSA+PMB und HPR10+PMB+HSA) wurden in Heparinplasma mit Endotoxinspike (5 ng/ml) inkubiert

Für den Endotoxinspike wurde *LPS Pseudomonas aeruginosa* (Fa. Sigma, L7018, Charge 128K4115) verwendet Die portionierten (à 100µl 10 ⁻³g/ml (1mg/ml)) und bei - 70 ° C in Mikrozentrifugenröhrchen gelagerten Endotoxine wurden aufgetaut und mit 900µl LAL-Wasser versetzt. Anschließend wurden die Endotoxine in NaCl bis zur erforderlichen Konzentration von *50 ng*/*ml* weiter verdünnt. Bei dem Ansatz des Tests erfolgte eine weitere Verdünnung der Endotoxinlösung bis zu einer Endkonzentration von 5 ng/ml.

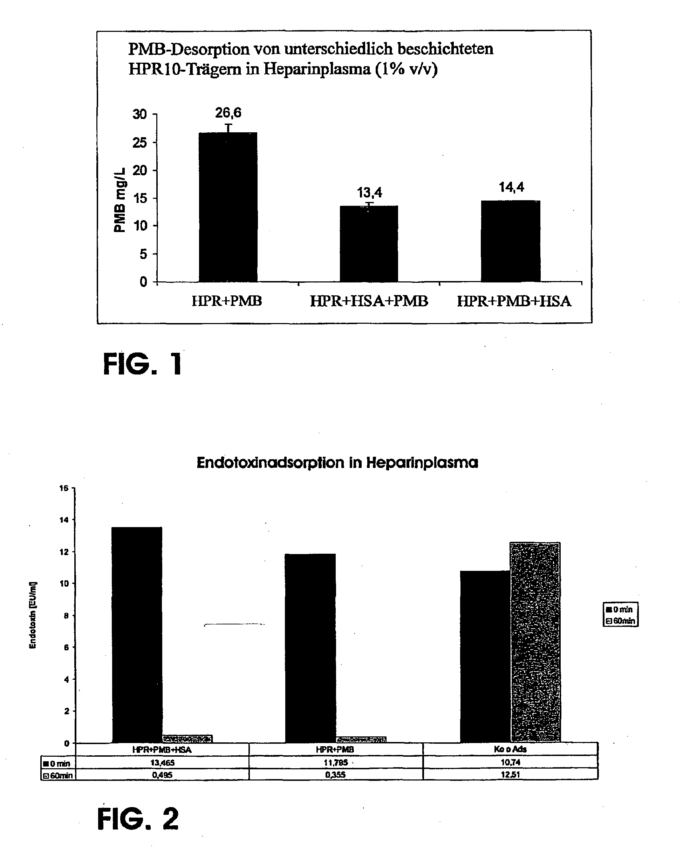

Es wurden nach 3, 6 und 24 Stunden Proben entnommen und die desorbierte PMB-Menge in Heparinplasma mittels PMB-Assay [Cao et al., 2008, Development and validation of reversed-phase high-performance liquid chromatography assay for polymyxin B in human plasma. Journal of Antimicrobial Chemotherapy 62:1009-1014] analysiert. Der Batchtest wurde mit zwei Wiederholungen durchgeführt. Die durchschnittliche PMB-Desorption betrug nach 24 h bei HPR10-PMB 26,6 mg/l, bei HPR10-HSA-PMB 13,4 mg/l und bei HPR10-PMB-HSA 14,4 mg/L. (siehe Fig. 1). Die Desorption des PMB von der Oberfläche des Adsorbers kann mit dem mit PMB und HSA beschichteten Sorptionsmittel gemäß der Erfindung (HPR+HSA+PMB und HPR+PMB+HSA) im Vergleich zu einem Sorptionsmittel, welches nur mit Polymyxin beschichtet ist (HPR10+PMB) um 45 % verringert werden.

Die Fähigkeit der Endotoxinadsorption, wird durch die zusätzliche Beschichtung mit Albumin nicht beeinträchtigt (siehe Fig. 2).

Zusammengefasst, weist das erfindungsgemäße Sorptionsmittel eine ebenso hohe Endotoxinsorptionseffizienz- und geschwindigkeit auf, wie ein Sorptionsmittel, welches nur mit Polymyxin beschichtet ist. Die zusätzliche Albuminbeschichtung bewirkt jedoch eine signifikante Verringerung der Desorption des toxischen Polymyxin B vom Träger und erhöht somit die Sicherheit für den Patienten.

### 3. BEISPIEL 3: Adsorption von Heparin und Protein C

### 3.1. Hintergrund des Versuchs

Viele Adsorber haben den Nachteil, dass nicht nur die zu entfernenden Substanzen, in diesem Fall Endotoxine, sondern auch physiologisch wichtige Proteine bzw. Faktoren des Blutgerinnungssytems (Antikoagulanzien sowie Prokoagulanzien) in sehr hohem Maße eliminiert werden. Hier sind insbesondere patienteneigene Gerinnungshemmer wie Protein C und Protein S zu nennen. Von besonderer Wichtigkeit ist das Protein C als gerinnungshemmender Faktor. Eine Verminderung der intrakorporalen Protein C-Konzentration führt zu schweren Gerinnungskomplikationen wie Venenthrombosen und Lungenembolien. Auch das per Infusion zusätzlich in den extrakorporalen Blutkreislauf zugeführte Antikoagulans Heparin wird durch viele Adsorber eliminiert. Die Folge ist ein Ungleichgewicht des intrakorporalen Gerinnungssystems bzw. eine unerwünschte Antikoagulation im extrakorporalen bzw. intrakorporalen Blutkreislauf, was eine große Gefahr für den Patienten bedeutet.

Die Versuche in diesem Beispiel 3 dienten somit dazu, das erfindungsgemäße neurartige Sorptionsmittel hinsichtlich der ungewollten Adsorption von Heparin und Protein C zu untersuchen. Verglichen wurden zwei verschiedene Träger unterschiedlicher Partikelgröße (#1824/5µm und HPR10/10µm - siehe Beispiel 1) bei unterschiedliche Beschichtungen (unbeschichtet/nur mit Polymyxin B beschichtet /mit Polymyxin B und Humanserumalbumin beschichtet). Die Heparinadsorption wurde in Heparinplasma (mit Heparin versetztes Blutplasma) untersucht. Die Adsorption von Protein C wurde in Citrat-Plasma (mit Citrat versetztes Blutplasma) untersucht.

### 3.2. Verwendete Adsorber

Es wurden Adsorber mit unterschiedlicher Beschichtung miteinander verglichen:
- unbeschichtete Träger (#1824 und HPR10),
- nur mit Polymyxin B beschichtete Träger (#1824+PMB und HPR10+PMB), und
- erfindungsgemäß mit Polymyxin B und Albumin beschichtete Träger (#1824+PMB+HSA und HPR10+PMB+HSA).

50%ige Adsorbersuspensionen der erfindungsgemäßen Adsorber #1824+PMB+HSA und HPR10+PMB+HSA wurden wie oben im Herstellungsprotokoll in Abschnitt 1.2. hergestellt.

Die Herstellung von 50%igen Adsorbersuspensionen der nur mit Polymyxin B beschichteten Adsorber erfolgte laut den Schritten a) - f) gemäß dem Herstellungsprotokoll in Beispiel 1.

Die in diesem Beispiel 3 verwendeten Adsorber sind in der Tabelle 3 zusammengefasst:

**Tabelle 3:**

| Bezeichnung des Adsorbers | Beschreibung des Adsorber |
|---|---|
| #1824 | unbeschichteter Microadsorber |
| #1824+PMB | Mit Polymyxin B beschichteter Microadsorber |
| #1824+PMB+HSA | Zuerst mit Polymyxin Bund anschließend mit HSA beschichteter Microadsorber |
| HPR10 | unbeschichtetes Amberchrom® HPR10 |
| HPR10+PMB | Mit Polymyxin B beschichtetes Amberchrom® HPR10 |
| HPR10+PMB+HSA | Zuerst mit Polymyxin B und anschließend mit HSA beschichtetes Amberchrom® HPR10 |

Als Kontrolle wurde ein Teströhrchen mit Plasma ohne Adsorber mitgeführt (Ko o Ads).

### 3.3. Blutplasma

a) HEPARIN-Plasma (soll:10 IU Heparin pro ml Plasma)
b) CITRAT-Plasma (soll: 11 mmol Citrat pro 1 Plasma)

### 3.4. Batchtests

Es wurden zwei Batchtests A und B durchgeführt, wobei für die Testansätze 50%ige Adsorbersuspensionen der verschiedenen Adsorber mit Heparin-Plasma (Batchtest A) und mit Citrat-Plasma (Batchtest B) in Teströhrchen inkubiert wurden, wobei jeweils 1470 ml Plasma mit 30 ml Adsorbersuspension versetzt wurden.

Die Adsorption von Heparin (in Heparin-Plasma) und Protein C (in Citrat-Plasma) wurde im Zeitverlauf bei 0,15 und 60 Minuten analysiert:

### Probennahme nach 15 und 60 Minuten:

Nach 15 min werden 500 µl Probe entnommen und bei 4000 g 5 min abzentrifugiert und aliquotiert: 1 x 100 µl (Protein C), 1 x 250 µl (Heparin), 1 x Rest (ca. 150 µl). Die Aliquote werden bei - 20 ° C bis zur Analytik gelagert.

Nach 60 min werden 500 µl Probe entnommen und bei 4000 g 5 min abzentrifugiert und aliquotiert: 1 x 100 µl (Protein C), 1 x 250 µl (Heparin), 1 x Rest (ca. 150 µl). Die Aliquote werden bei - 20 ° C bis zur Analytik gelagert.

### 3.5. Ergebnisse der Batchtests

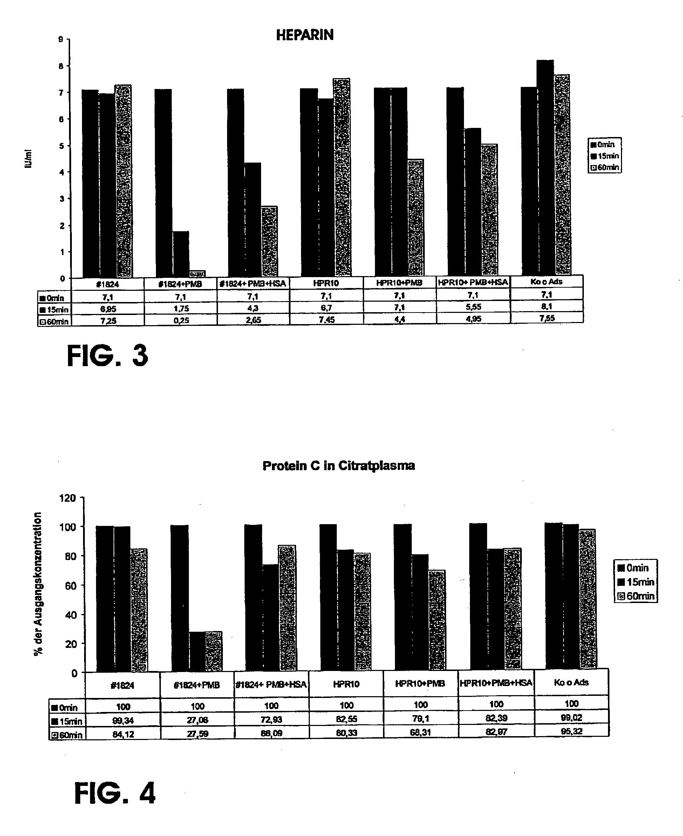

Die Ergebnisse der Batchtests sind in den Figuren 3 und 4 dargestellt:
Fig. 3 zeigt die Adsorption des Heparins durch die verschiedenen Adsorber in Heparin-Plasma. Es hat sich überraschenderweise gezeigt, dass die unerwünschte Adsorption des Heparins durch Verwendung eines erfindungsgemäßen, mit Polymyxin B und HSA beschichteten Sorptionsmittels (Adsorbers) - insbesondere beim Adsorber geringer Partikelgröße (5 µm Partikelgröße) - verringert werden kann.
Fig. 4 zeigt die Adsorption von Protein C durch die verschiedenen Adsorber in Citrat-Plasma. Es hat sich gezeigt, dass Protein C von den erfindungsgemäßen Adsorbern kaum adsorbiert wird und die Protein-C-Adsorption im Vergleich zu den anderen Adsorbern (unbeschichtet bzw. nur mit Polymyxin B beschichtet) im Wesentlichen gleich blieb (HPR10+PMP+HSA - 10 µm Partikeldurchmesser) bzw. sogar deutlich verringert wurde (#1824+PMB+HSA - 5 µm Partikeldurchmesser).

## Patentansprüche

1. Sorptionsmittel zum Entfernen von Endotoxinen aus einer biologischen Flüssigkeit, umfassend einen wasserunlöslichen, porösen Träger mit einer nicht-ionischen, hydrophoben Oberfläche, wobei die Oberfläche des Trägers eine adsorptive Beschichtung aus Polymyxin und Albumin aufweist, wobei Polymyxin und Albumin nichtkovalent mit der Oberfläche des Trägers verbunden sind.

2. Sorptionsmittel nach Anspruch 1, **dadurch gekennzeichnet, dass** der Träger ein hydrophobes Polymer ist.

3. Sorptionsmittel nach Anspruch 1 oder 2, **dadurch gekennzeichnet, dass** das hydrophobe Polymer ein vernetztes Polystyrol-Polymer, insbesondere ein Polystyrol-Divinylbenzol-Copolymer ist.

4. Sorptionsmittel nach einem der Ansprüche 1 bis 3, **dadurch gekennzeichnet, dass** der Träger eine mittlere Porengröße von zumindest 15 nm, vorzugsweise von zumindest 30 nm aufweist, wobei sich die mittlere Porengröße auf den mittleren Durchmesser der Poren bezieht.

5. Sorptionsmittel nach einem der Ansprüche 1 bis 4, **dadurch gekennzeichnet, dass** der Träger eine mittlere Porengröße von nicht mehr als 120 nm aufweist, wobei sich die mittlere Porengröße auf den mittleren Durchmesser der Poren bezieht.

6. Sorptionsmittel nach einem der Ansprüche 1 bis 6, **dadurch gekennzeichnet, dass** das Sorptionsmittel die Form von Mikropartikeln hat.

7. Sorptionsmittel nach Anspruch 6, **dadurch gekennzeichnet, dass** die Mikropartikel eine Partikelgröße von 20 µm oder kleiner, vorzugsweise eine Partikelgröße von 8 µm oder kleiner, idealerweise 5 µm oder kleiner, aufweisen.

8. Sorptionseinrichtung, enthaltend ein Sorptionsmittel nach einem der Ansprüche 1 bis 7.

9. Blutplasmakreislauf, enthaltend eine Suspension eines Sorptionsmittels nach einem der Ansprüche 1 bis 7.

10. Verfahren zum Herstellen eines Sorptionsmittels gemäß einem der Ansprüche 1 bis 7, die Schritte umfassend:
a) Bereitstellen eines wasserunlöslichen, porösen Trägers mit einer nicht-ionischen, hydrophoben Oberfläche,
b) Adsorptives Beschichten der Oberfläche des Trägers mit Polymyxin durch Inkontaktbringen und Inkubieren des Trägers mit einer wässrigen, Polymyxin enthaltenden Lösung,
c) Waschen des Trägers zum Entfernen von ungebundenem Polymyxin,
d) Adsorptives Beschichten der mit Polymyxin beschichteten Oberfläche des Trägers mit Albumin durch Inkontaktbringen und Inkubieren des Trägers mit einer wässrigen, Albumin enthaltenden Lösung, und
e) gegebenenfalls Waschen des Trägers zum Entfernen von ungebundenem Albumin.

11. Verfahren nach Anspruch 10, **dadurch gekennzeichnet, dass** der Träger zwischen den Schritten b) und c) oder zwischen den Schritten c) und d) autoklaviert wird.

12. Sorptionsmittel nach einem der Ansprüche 1 bis 7 zur Behandlung einer Sepsis.

13. Microspheres-based Detoxification System (MDS) umfassend ein Sörptionsmittel nach einem der Ansprüche 1 bis 7.

## Claims

1. A sorbent for removing endotoxins from a biological fluid, comprising a water-insoluble, porous carrier having a non-ionic, hydrophobic surface, wherein the surface of the carrier has an adsorptive coating formed from polymyxin and albumin, wherein polymyxin and albumin are bonded to the surface of the carrier in a non-covalent manner.

2. The sorbent according to Claim 1, **characterised in that** the carrier is a hydrophobic polymer.

3. The sorbent according to Claim 1 or 2, **characterised in that** the hydrophobic polymer is a cross-linked polystyrene polymer, in particular a polystyrene-divinyl benzene copolymer.

4. The sorbent according to one of Claims 1 to 3, **characterised in that** the carrier has a mean pore size of at least 15 nm, preferably of at least 30 nm, wherein the mean pore size relates to the mean diameter of the pores.

5. The sorbent according to one of Claims 1 to 4, **characterised in that** the carrier has a mean pore size of no more than 120 nm, wherein the mean pore size relates to the mean diameter of the pores.

6. The sorbent according to one of Claims 1 to 6, **characterised in that** the sorbent has the form of microparticles.

7. The sorbent according to Claim 6, **characterised in that** the microparticles have a particle size of 20 µm or less, preferably a particle size of 8 µm or less, ideally 5 µm or less.

8. A sorption apparatus containing a sorbent according to one of Claims 1 to 7.

9. A blood plasma circuit, containing a suspension of a sorbent according to one of Claims 1 to 7.

10. A method for producing a sorbent according to one of Claims 1 to 7, comprising the steps of:
a) providing a water-insoluble, porous carrier having a non-ionic, hydrophobic surface,
b) adsorptively coating the surface of the carrier with polymyxin by contacting and incubating the carrier with an aqueous polymyxin-containing solution,
c) washing the carrier to remove any unbound polymyxin,
d) adsorptively coating the polymyxin-coated surface of the carrier with albumin by contacting and incubating the carrier with an aqueous albumin-containing solution, and
e) as appropriate, washing the carrier to remove any unbound albumin.

11. The method according to Claim 10, **characterised in that** the carrier is autoclaved between steps b) and c) or between steps c) and d).

12. The sorbent according to one of Claims 1 to 7 for the treatment of sepsis.

13. A microspheres-based detoxification system (MDS) comprising a sorbent according to one of Claims 1 to 7.

## Revendications

1. Agent de sorption destiné à éliminer les endotoxines d'un liquide biologique, comprenant un support poreux insoluble dans l'eau avec une surface hydrophobe non ionique, dans lequel la surface du support présente un revêtement adsorptif constitué de polymyxine et d'albumine, dans lequel la polymyxine et l'albumine sont liées de manière non covalente avec la surface du support.

2. Agent de sorption selon la revendication 1, **caractérisé en ce que** le support est un polymère hydrophobe.

3. Agent de sorption selon la revendication 1 ou 2, **caractérisé en ce que** le polymère hydrophobe est un polymère de polystyrène réticulé, en particulier un copolymère de polystyrène - divinylbenzène.

4. Agent de sorption selon une des revendications 1 à 3, **caractérisé en ce que** le support présente une taille moyenne de pores d'au moins 15 nm, de préférence d'au moins 30 nm, dans lequel la taille moyenne des pores se rapporte au diamètre moyen des pores.

5. Agent de sorption selon une des revendications 1 à 4, **caractérisé en ce que** le support présente une taille moyenne de pores qui n'excède pas 120 nm, dans lequel la taille moyenne des pores se rapporte au diamètre moyen des pores.

6. Agent de sorption selon une des revendications 1 à 6, **caractérisé en ce que** l'agent de sorption prend la forme de microparticules.

7. Agent de sorption selon la revendication 6, **caractérisé en ce que** les microparticules présentent une taille de particules de 20 µm ou moins, de préférence une taille de particules de 8 µm ou moins, idéalement de 5 µm ou moins.

8. Dispositif de sorption, comprenant un agent de sorption selon une des revendications 1 à 7.

9. Circuit de plasma sanguin, comprenant une suspension d'un agent de sorption selon une des revendications 1 à 7.

10. Procédé de préparation d'un agent de sorption selon une des revendications 1 à 7, comprenant les étapes de :
a) mise à disposition d'un support poreux insoluble dans l'eau avec une surface hydrophobe non ionique,
b) dépôt d'un revêtement adsorptif sur la surface du support comprenant de la polymyxine par mise en contact et incubation du support avec une solution aqueuse contenant de la polymyxine,
c) lavage du support pour éliminer la polymyxine non liée,
d) dépôt d'un revêtement adsorptif sur la surface du support revêtue de polymyxine comprenant de l'albumine par mise en contact et incubation du support avec une solution aqueuse contenant de l'albumine, et
e) le cas échéant lavage du support pour éliminer l'albumine non liée.

11. Procédé selon la revendication 10, **caractérisé en ce que** le support est autoclavé entre les étapes b) et c) ou entre les étapes c) et d).

12. Agent de sorption selon une des revendications 1 à 7 pour le traitement d'une septicémie.

13. Système de Détoxification à base de Microsphères (SDM) comprenant un agent de sorption selon une des revendications 1 à 7.
